# EUROPÄISCHE PATENTSCHRIFT

(11) **EP 3 290 409 B1**
(45) Veröffentlichungstag und Bekanntmachung des Hinweises auf die Patenterteilung: **07.08.2019**
(21) Anmeldenummer: 16186941.7
(22) Anmeldetag: 02.09.2016
(51) Int. Cl.: C07D 301/12

(54) **VERFAHREN ZUR HERSTELLUNG VON 17-OXABICYCLO[14.1.0]HEPTADEC-8-EN**
PROCESS FOR THE PREPARATION OF 17-OXABICYCLO[14.1.0]HEPTADEC-8-EN
PROCEDE POUR LA PREPARATION DE 17-OXABICYCLO[14.1.0]HEPTADEC-8-EN

(43) Veröffentlichungstag der Anmeldung: 07.03.2018
(73) Patentinhaber: Symrise AG, 37603 Holzminden (DE)
(72) Erfinder: Esser, Peter, 37639 Bevern (DE); Köckritz, Angela, 12437 Berlin (DE); Martin, Andreas, 12524 Berlin (DE); Jaime, Diego, 18057 Rostock (DE)
(74) Vertreter: Global IP Europe Patentanwaltskanzlei

(56) Entgegenhaltungen:
- EP-A1- 0 109 273
- EP-B1- 0 434 546
- WO-A1-2007/090704
- DE-A1- 2 111 753
- MOOKHERJEE B D ET AL: "Synthesis of racemic muscone and cyclopentadecanone (exaltone) from 1,9-cyclohexadecadiene", THE JOURNAL OF ORGANIC CHEMISTRY, AMERICAN CHEMICAL SOCIETY, US, Bd. 36, Nr. 22, 1. Januar 1971 (1971-01-01), Seiten 3266-3270, XP002432756, ISSN: 0022-3263, DOI: 10.1021/JO00821A003
- KACZMARCZYK E ET AL: "Epoxidation of 1,4-diallyloxybutane to 1-allyloxy-4-glycidyloxybutane by the method of phase transfer catalysis", JOURNAL OF MOLECULAR CATALYSIS A: CHEMICAL, ELSEVIER, AMSTERDAM, NL, Bd. 235, Nr. 1-2, 1. Juli 2005 (2005-07-01), Seiten 52-56, XP027658191, ISSN: 1381-1169 [gefunden am 2005-07-01]
- PETER T. WITTE ET AL: "Self-Assembled Na 12 [WZn 3 (ZnW 9 O 34 ) 2 ] as an Industrially Attractive Multi-Purpose Catalyst for Oxidations with Aqueous Hydrogen Peroxide", ORGANIC PROCESS RESEARCH AND DEVELOPMENT, Bd. 8, Nr. 3, 1. Mai 2004 (2004-05-01), Seiten 524-531, XP55312563, US ISSN: 1083-6160, DOI: 10.1021/op034184o

## Beschreibung

### Gebiet der Erfindung

Die vorliegende Erfindung betrifft ein Verfahren zur Herstellung von 17-Oxabicyclo[14.1.0]heptadec-8-en aus Cyclohexadeca-1,9-dien (CHDD).

### Stand der Technik

17-Oxabicyclo[14.1.0]heptadec-8-en ist eine Zwischenstufe des Moschusduftstoffs 8-Cyclohexadecen-1-on und kann durch bereits bekannte Herstellverfahren dargestellt werden. DE 2111753 und DE 112007000301 offenbaren jeweils die Herstellung von 17-Oxabicyclo[14.1.0]heptadec-8-en aus Cyclohexadeca-1,9-dien mit Persäuren.

WO 2007/090704 A1 offenbart die Herstellung von 17-Oxabicyclo[14.1.0]heptadec-8-en aus Cyclohexadeca-1,9-dien unter Verwendung von Peressigsäure.

Mookherjee B. D. et al.: "Synthesis of racemic muscone and cyclopentadecanone (exaltone) from 1,9-cyclohexydecadiene", The Journal of Organic Chemistry, American Chemical Society, US, Bd. 36, Nr. 22, 1. Januar 1971 (1971-01-01), Seiten 3266 - 3270, XP002432756, ISSM: 0022-3263, DOI: 10.1021/JO00821 A003, offenbart die Herstellung von Muscon aus Cyclohexadeca-1,9-dien unter Verwendung von Peressigsäure.

EP 0 434 546 B1 betrifft ein Verfahren zur seletiven Epoxidation von ungesättigten (Meth)acrylverbindungen in Gegenwart eines Alkalimolybdat- oder -wolframat-Katalysators.

Kaczmarczyk E. et al.: "Epoxidation of 1,4-diallyloxybutane to 1-allyloxy-4-glycidyloxybutane by the method of phase transfer catalysis", Journal of Molecular Catalysis, A Chemical, Elsevier, Amsterdam, NL, Bd. 235, Nr. 1 - 2. 1. Juli 2005 (2005-07-01), Seiten 52 - 56, XP027658191, ISSN 1381-1169, offenbart die Epoxidierung von 1,4-Diallyloxybutan mittels Wasserstoffperoxid in Gegenwart eines Phasentransfer-Katalysators.

EP 0 109 273 A1 betrifft die Herstellung einer Peroxidzusammensetzung, bestehend aus einem Peroxyphosphorwolframat und/oder einem Peroxyarsenowolframat und dessen Verwendung bei der Epoxidierung von olefinischen Verbindungen.

Peter T. Witte et al.: "Self-assembled Na12[WZn3(ZnW9O34)2] as an industrially attractive multi-purpose catalyst for oxidations with aqueous hydrogen peroxide", Organic Process Research and Development, Bd. 8, Nr. 3, 1. Mai 2004 (2004-05-01), Seiten 524 - 531, XP55312563, US, USSN: 1083-6160, DOI: 10.1021/op034184o, offenbart die Epoxidierung von Alkenen mit Wasserstoffperoxid.

### Aufgabe der Erfindung

Es ist die Aufgabe der vorliegenden Erfindung, Cyclohexadeca-1,9-dien selektiv an einer Doppelbindung zu 17-Oxabicyclo[14.1.0]heptadec-8-en zu epoxidieren. Die Reaktion soll wirtschaftlich, mit hoher Ausbeute, unter nachhaltigen Bedingungen, hoher Selektivität, geringerem Energieeinsatz, geringerem Rohstoffverbrauch, wenig Nebenprodukten, mit hoher Reaktionsgeschwindigkeit, mit geringer Anlagenkorrosion, atomökonomisch und umweltfreundlich durchzuführen sein. Insbesondere soll die Bildung von unerwünschten Diepoxiden vermieden oder minimiert werden, u.a. weil diese schwer von den erwünschten Monoepoxiden getrennt werden können und der Aufwand für die Trennung erheblich ist.

### Beschreibung der Erfindung

Das erfindungsgemäße Verfahren zur Herstellung von 17-Oxabicyclo[14.1.0]heptadec-8-en umfasst eine Reaktion, in der Cyclohexadeca-1,9-dien und Wasserstoffperoxid als Reaktanten verwendet werden und die Reaktion in Gegenwart eines Katalystors durchgeführt wird.

Bevorzugt beträgt das molekulare Verhältnis von Cyclohexadeca-1,9-dien zu Wasserstoffperoxid 1 : kleiner als 1, weiter bevorzugt 1 : 0,1-0,9 und besonders bevorzugt 1 : 0,4-0,6.

Cyclohexadeca-1,9-dien und dessen Herstellung sind bereits bekannt und es ist auch kommerziell verfügbar. Es liegt oft als Stereoisomerengemisch vor.

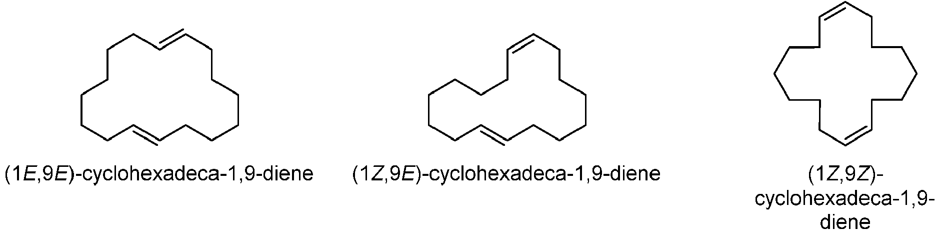

Wasserstoffperoxid (H₂O₂) und dessen Herstellung sind ebenfalls bekannt und es ist ebenfalls kommerziell verfügbar.

Ein weiterer Vorteil des Verfahrens ist, dass keine zwingende Notwendigkeit besteht, halogenhaltige Lösungsmittel bei der Reaktion einzusetzen, so dass die Reaktion ohne halogenhaltige Lösungsmittel, insbesondere chlorhaltige Lösungsmittel, durchgeführt werden kann. Insofern erübrigt sich die Entsorgung der halogenhaltigen Lösungsmittel und es besteht nicht, die Gefahr, dass sich unerwünschte halogenorganische Verbindungen bilden. Bevorzugt sind halogenfreie Lösungsmittel wie aliphatische oder cyclische Kohlenwasserstoffe und alkylierte Aromaten.

Die Reaktion von Cyclohexadeca-1,9-dien und Wasserstoffperoxid kann in einem Zweiphasensystem durchgeführt werden. Dieses lässt sich zum Beispiel dadurch erreichen, dass den Reaktanten entweder keine Lösungsmittel oder nur sehr unpolare (wie Toluen) bzw. sehr polare Lösungsmittel (wie Wasser) hinzugefügt werden.

In dem erfindungsgemäßen Verfahren wird ein Katalysator eingesetzt, wobei insbesondere phosphorenthaltende oder/und wolframenthaltende Katalysatoren geeignet sind. Ferner ist auch der Einsatz eines Phasentransferkatalysators vorteilhaft.

Bevorzugt lässt man den Katalysator einschließlich deren aktive Spezies in situ aus Katalysatorpräkursoren entstehen. Einer der Vorteile der in situ Bildung besteht darin, dass die aktive Spezies im Vergleich zur ex situ Bildung nicht isoliert werden muss, um in dem Verfahren eingesetzt werden zu können. Phosphorenthaltende Katalysatorpräkursoren sind z.B. Phosphorsäure, Phosphonsäuren wie Hydroxymethanphosphonsäure und Aminomethanphosphonsäure, Phosphinsäuren wie Diphenylphosphinsäure oder Di(hydroxymethan)phosphinsäure und Heteropolysäuren wie Wolframatophosphorsäure oder Molybdatophosphorsäure und deren Derivate (z.B. lacunare Heteropolysäuren und Polyoxometallate). Möglich ist auch eine Variation des Präkursors der Phosphorkomponente. So sind Phosphonsäuren neben H₃PO₄ sehr gut geeignet. Besonders bevorzugt sind dabei Hydroxymethanphosphonsäure und Phenylphosphonsäure.

Wolframenthaltende Katalysatorpräkursoren sind z.B. wasserlösliche Wolframverbindungen, Wolframate, Wolfram(VI)-verbindungen, Alkaliwolframate, Erdalkaliwolframate, Ammoniumwolframate oder Wolframtrioxid-monohydrat. Na₂WO₄ ist ein konkretes Beispiel eines wolframenthaltenden Katalysatorpräkursors. Beispiele eines Phasentransferkatalysators sind Tetraalkylammoniumsalz(e) oder bevorzugt eine oder mehrere Verbindungen der Formel

(R¹ₙR²ₘN⁺)_{y}X^{y-},

dadurch gekennzeichnet, dass
R¹ und R² jeweils C1-C30 n-Alkyl bedeuten und R¹ gleich oder verschieden zu R² ist, sowie die Summe aus m und n gleich 4 ist,
X^{y-} gleich Cl⁻, Br⁻, J⁻, HSO₄⁻, SO₄²⁻, H₂PO₄⁻, HPO₄²⁻, PO₄³⁻, CH₃SO₃⁻, CF₃SO₃⁻, CH₃C₆H₄SO₃⁻, ClO₃⁻, ClO₄⁻ oder NO₃⁻ ist und die Summe aus m und n gleich 4 ist und y gleich 1, 2 oder 3 ist.

Bevorzugte Anionen des Phasentransferkatalysators sind Hydrogensulfatanionen, Sulfonsäureanionen oder Dihydrogenphosphatanionen, besonders bevorzugt sind Hydrogensulfatanionen.

Ein Beispiel für einen Phasentransferkatalysator ist Aliquat 336 ® (Methyltrioctylammoniumchlorid).

Es ist vorteilhaft, wenn sich am Ammoniumstickstoff 1 bis 3 Methylgruppen befinden, wobei dann die verbleibenden Alkylgruppen am Ammoniumstickstoff eine größere Kettenlänge zwischen 6 und 30 Kohlenstoffatomen in der Kette aufweisen sollten, eine bevorzugte Kettenlänge liegt zwischen 8 und 22 Kohlenstoffatomen.

Beim Mischen der wolfram- und phosphatenthaltenden Katalysatorpräkursoren in Gegenwart von Wasserstoffperoxid und Wasser entstehen Peroxowolframatophosphate. Es wird angenommen, dass viele geeignete Peroxowolframatophosphate über das Anion {PO₄[WO(O₂)₂]₄}³⁻verfügen.
Der kationische Teil der aktiven Spezies des Katalysators kann gebildet werden vom Kation eines Phasentransferkatalysators, insbesondere kann das Kation des Phasentransferkatalysators die Formel

R¹ₙR²ₘN⁺

aufweisen, dadurch gekennzeichnet, dass
R¹ und R² jeweils C1-C30 n-Alkyl bedeuten und R¹ gleich oder verschieden zu R² ist, sowie die Summe aus m und n gleich 4 ist.

Zur Herstellung der aktiven Spezies des Katalysators kann eine wässrige Mischung /Lösung umfassend mindestens eine phosphorenthaltende Säure, mindestens eine Wolfram (VI)-Verbindung und mindestens einen Phasentransferkatalysator sowie gegebenenfalls Wasserstoffperoxid verwendet werden. Tabelle A enthält Beispiele für die wolframenthaltenden und phosphorenthaltenden Katalysatorpräkursoren und Phasentransferkatalysatoren solcher wässrigen Mischungen.

**Tabelle A**

| Bsp. Nr. | wolframenthaltender Katalysatorpräkursor | phosphorenthaltender Katalysatorpräkursor | Phasentransferkatalysator |
|---|---|---|---|
| 1 | Na₂WO₄ | HOCH₂P(O)(OH)₂ | [CH₃(C₈H₁₇)₃N]Cl |
| 2 | Na₂WO₄ | HOCH₂P(O)(OH)₂ | [CH₃(C₈H₁₇)₃N]HSO₄ |
| 3* | Na₂WO₄ | HOCH₂P(O)(OH)₂ | [CH₃(C₁₈H₃₇)₃N]HSO₄ |
| 4 | Na₂WO₄ | C₆H₅P(O)(OH)₂ | [CH₃(C₈H₁₇)₃N]Cl |
| 5 | Na₂WO₄ | C₆H₅P(O)(OH)₂ | [CH₃(C₈H₁₇)₃N]HSO₄ |
| 6* | Na₂WO₄ | C₆H₅P(O)(OH)₂ | [CH₃(C₁₈H₃₇)₃N]HSO₄ |
| 7 | Na₂WO₄ | H₂NCH₂P(O)(OH)₂ | [CH₃(C₈H₁₇)₃N]Cl |
| 8 | Na₂WO₄ | H₂NCH₂P(O)(OH)₂ | [CH₃(C₈H₁₇)₃N]HSO₄ |
| 9 | Na₂WO₄ | H₃PO₄ | [(C₄H₉)₄N]HSO₄ |
| 10 | Na₂WO₄ | H₃PO₄ | [CH₃(C₈H₁₇)₃N]Cl |
| 11 | Na₂WO₄ | H₃PO₄ | [CH₃(C₈H₁₇)₃N]HSO₄ |
| 12* | Na₂WO₄ | H₃PO₄ | [(CH₃)₂(C₁₈H₃₇)₂N]HSO₄ |
| 13* | Na₂WO₄ | H₃PO₄ | [(C₁₈H₃₇)₄N]HSO₄ |
| 14 | Na₂WO₄ | H₃PO₄ | [(CH₃)₃(C₁₆H₃₃)N]O₃SC₆H₄-4-CH₃ |
| 15* | Na₂WO₄ | H₃PO₄ | [CH₃(C₈H₁₇)₃N]H₂PO₄ |
| 16 | Na₂WO₄ | (C₆H₅)₂P(O)OH | [CH₃(C₈H₁₇)₃N]HSO₄ |
| 17 | Na₂WO₄ | H₂NCH₂P(O)(OH)₂ | [CH₃(C₈H₁₇)₃N]HSO₄ |
| 18 | Na₂WO₄ | H₃PO₄ | [CH₃(C₈H₁₇)₃N]HSO₄ |
| 19 | Na₂WO₄ | HOCH₂P(O)(OH)₂ | [CH₃(C₈H₁₇)₃N]HSO₄ |
| 20 | Na₂WO₄ | (HOCH₂)₂P(O)OH | [CH₃(C₈H₁₇)₃N]HSO₄ |
| 21 | Na₂WO₄ | (HOCH₂)₂P(O)OH | [CH₃(C₈H₁₇)₃N]HSO₄ |

Die Erfindung umfasst auch eine oder mehrere Verbindungen der Formel

[R¹ₙR²ₘN⁺]₃{PO₄[WO(O₂)₂]₄}

dadurch gekennzeichnet, dass
R¹ und R² jeweils C1-C30 n-Alkyl bedeuten und R¹ gleich oder verschieden zu R² ist, sowie die Summe aus m und n gleich 4 ist.

Diese Verbindungen können als aktive Spezies eines Katalysators in dem erfindungsgemäßen Verfahren eingesetzt werden und entstehen beim Mischen der bereits genannten Katalysatorpräkursoren und Phasentransferkatalysatoren in Wasser in Gegenwart von Wasserstoffperoxid. Beispiele für diese Verbindungen sind

[CH₃(C₈H₁₇)₃N]₃{PO₄[WO(O₂)₂]₄},

[(CH₃)₂(C₈H₁₇)₂N]₃{PO₄[WO(O₂)₂]₄},

[CH₃(C₁₈H₃₇)₃N]₃{PO₄[WO(O₂)₂]₄},

[(C₄H₉)₄N]₃{PO₄[WO(O₂)₂]₄},

[(CH₃)₂(C₁₈H₃₇)₂N]₃{PO₄[WO(O₂)₂]₄},

[(C₁₈H₃₇)₄N]₃{PO₄[WO(O₂)₂]₄}

und

[(CH₃)₃(C₁₆H₃₃)N]₃{PO₄[WO(O₂)₂]₄}.

Das erfindungsgemäße Verfahren kann zudem einen Trennschritt, wie eine Separation der Phasen, Destillation oder / und eine chromatografische Trennung umfassen.

Das Verfahren kann diskontinuierlich oder kontinuierlich durchgeführt werden.
Die folgenden Beispiele erläutern die Erfindung, stellen aber keine Einschränkung dar.

### Allgemeine Vorschrift für Beispiele 1-15 (Tabelle 1)

Na₂WO₄ (0,165 g, 0,50 mmol), H₃PO₄ oder eine der genannten Phosphonsäuren (0,50 mmol) und ein Phasentransferkatalysator (0,50 mmol) wurden in einen 50-ml-Dreihalskolben gegeben. 1,9-Cyclohexadecadien (Isomerengemisch, 25 mmol, 5,51 g), H₂O (5,00 g) und Toluen (20,00 g) wurden anschließend hinzugefügt. Es bildeten sich zwei Phasen, eine organische Phase, welche aus Toluol und CHDD bestand, und eine wässrige Phase, welche die Präkursoren für den Katalysator enthielt. Das Gemisch wurde dann bei 800 rpm gerührt und auf die Reaktionstemperatur von 60 °C aufgeheizt. Als diese Temperatur erreicht war, wurde die erste Portion H₂O₂ (50 Gew.%) (0,47 g, 6,91 mmol, 0,27 Moläqu.) zugegeben und die Reaktion gestartet. Nach 60 min wurde eine zweite Portion H₂O₂ zugetropft (0.47 g, 6,91 mmol, 0,27 Moläqu.). Danach wurde noch 2 Stunden bei 60 °C weiter gerührt. Der Fortgang der Reaktion wurde durch Probennahme aus der organischen Phase alle 20 Minuten während der ersten zwei Stunden und am Ende des Experiments überprüft. Die Ermittlung von Umsatz, Ausbeuten und Selektivitäten erfolgte mittels GC/MS.

**Tabelle 1**

| Beispiele | Phosphorkomponente | Phasentransferkatalysator | t [min] | Umsatz von CHDD [%] | Ausbeute an **I** [%] | Selektivität zu **I** [%] |
|---|---|---|---|---|---|---|
| 1 | HOCH₂P(O)(OH)₂ | [CH₃(C₈H₁₇)₃N]Cl | 80 | 11,4 | 11,4 | 100 |
| | | | 100 | 16,3 | 15,9 | 97,3 |
| | | | 180 | 30,1 | 27,7 | 92,1 |
| 2 | HOCH₂P(O)(OH)₂ | [CH₃(C₈H₁₇)₃N]HSO₄ | 20 | 11,8 | 11,8 | 100 |
| | | | 40 | 22,1 | 21,1 | 95,6 |
| | | | 60 | 22,7 | 21,7 | 95,2 |
| | | | 80 | 29,2 | 27,2 | 93,2 |
| 3* | HOCH₂P(O)(OH)₂ | [CH₃(C₁₈H₃₇)₃N]HSO₄ | 20 | 12,5 | 12,5 | 100 |
| | | | 40 | 21,6 | 20,1 | 93,3 |
| | | | 60 | 24,2 | 22,4 | 92,5 |
| 4 | C₆H₅P(O)(OH)₂ | [CH₃(C₈H₁₇)₃N]Cl | 100 | 14,8 | 14,8 | 100 |
| | | | 120 | 19,2 | 18,3 | 95,7 |
| | | | 180 | 25,7 | 23,8 | 92,7 |
| 5 | C₆H₅P(O)(OH)₂ | [CH₃(C₈H₁₇)₃N]HSO₄ | 60 | 19,3 | 18,5 | 95,6 |
| | | | 80 | 25,2 | 23,8 | 94,5 |
| | | | 100 | 31,6 | 29,0 | 91,7 |
| 6* | C₆H₅P(O)(OH)₂ | [CH₃(C₁₈H₃₇)₃N]HSO₄ | 60 | 19,4 | 18,3 | 94,3 |
| | | | 80 | 24,3 | 22,7 | 93,7 |
| | | | 100 | 31,3 | 28,8 | 92,1 |
| 7 | H₂NCH₂P(O)(OH)₂ | [CH₃(C₈H₁₇)₃N]Cl | 60 | 14,8 | 14,5 | 97,9 |
| | | | 80 | 23,3 | 22,4 | 96,2 |
| | | | 100 | 32,5 | 30,2 | 93,0 |
| 8 | H₂NCH₂P(O)(OH)₂ | [CH₃(C₈H₁₇)₃N]HSO₄ | 60 | 13,2 | 13,1 | 99,2 |
| | | | 80 | 23,8 | 22,5 | 94,6 |
| | | | 100 | 34,5 | 31,7 | 91,8 |
| 9 | H₃PO₄ | [(C₄H₉)₄N]HSO₄ | 120 | 0 | 0 | 0 |
| 10 | H₃PO₄ | [CH₃(C₈H₁₇)₃N]Cl | 20 | 15,8 | 13,2 | 83,6 |
| | | | 60 | 29,4 | 25,1 | 85,4 |
| | | | 100 | 42,1 | 34,4 | 81,8 |
| 11 | H₃PO₄ | [CH₃(C₈H₁₇)₃N]HSO₄ | 20 | 10,9 | 10,7 | 98,7 |
| | | | 40 | 23,8 | 22,7 | 95,3 |
| | | | 60 | 28,7 | 26,6 | 92,7 |
| 12* | H₃PO₄ | [(CH₃)₂(C₁₈H₃₇)₂N]HSO₄ | 20 | 10,1 | 10,1 | 100 |
| | | | 40 | 20,7 | 19,4 | 93,7 |
| | | | 80 | 37,8 | 34,5 | 91,4 |
| 13* | H₃PO₄ | [(C₁₈H₃₇)₄N]HSO₄ | 60 | 6,7 | 6,5 | 97,2 |
| | | | 80 | 14,4 | 14,0 | 97,4 |
| | | | 100 | 23,9 | 22,6 | 94,5 |
| | | | 120 | 31,1 | 28,4 | 91,2 |
| 14 | H₃PO₄ | [(CH₃)₃(C₁₆H₃₃)N]O₃S-C₆H₄-4-CH₃ | 60 | 12,0 | 12,0 | 100 |
| | | | 120 | 24,1 | 23,0 | 95,4 |
| | | | 180 | 33,7 | 31,1 | 92,1 |
| 15* | H₃PO₄ | [CH₃(C₈H₁₇)₃N]H₂PO₄ | 40 | 18,6 | 18,1 | 97,1 |
| | | | 60 | 23,5 | 22,4 | 95,3 |
| | | | 80 | 31,1 | 28,5 | 91,5 |
| | | | 100 | 37,1 | 33,8 | 90,9 |

| | | | | | | |
|---|---|---|---|---|---|---|
| * Für Beispiel 15 (Tabelle 1) wurde ein halber Ansatz mit folgenden Mengen gefahren: Na₂WO₄ (0,083 g, 0,25 mmol), H₃PO₄ (0,25mmol), PTC (0,25 mmol), 1,9-Cyclohexadecadien (2,75 g, 12,5 mmol), Toluen (10,0 g) und H₂O (2,5 g) sowie 2 Portionen von 50 Gew% H₂O₂ (0,24 g, 3,53 mmol, je 0,28 Moläqu.). Die Reaktionsdurchführung erfolgte genauso wie in der allgemeinen Vorschrift für die Beispiele 1-14 beschrieben. | | | | | | |

### Vorschrift für Beispiel 16 (Tabelle 2)

Na₂WO₄ (0,083 g, 0,25 mmol), Diphenylphosphinsäure (0,054 g, 0,25 mmol) und Methyltrioctylammoniumhydrogensulfat (0,25 mmol) wurden in einen 25-ml-Dreihalskolben gegeben. 1,9-Cyclohexadecadien (Isomerengemisch, 12,5 mmol, 2,75 g), H₂O (2,50 g) und Toluen (10,00 g) wurden anschließend hinzugefügt. Es bildeten sich zwei Phasen, eine organische Phase, welche aus Toluen und CHDD bestand, und eine wässrige Phase, welche die Präkursoren für den Katalysator enthielt. Das Gemisch wurde dann bei 800 rpm gerührt und auf die Reaktionstemperatur von 80 °C aufgeheizt. Als diese Temperatur erreicht war, wurde die erste Portion H₂O₂ (50 Gew.%) (0,24 g, 3,53 mmol, 0,28 Moläqu.) zugegeben und die Reaktion gestartet. Nach 60 min wurde eine zweite Portion H₂O₂ zugetropft (0,24 g, 3,53 mmol, 0,28 Moläqu.). Danach wurde noch 2 Stunden bei 80 °C weiter gerührt. Der Fortgang der Reaktion wurde durch Probennahme aus der organischen Phase alle 20 Minuten während der ersten zwei Stunden und am Ende des Experiments überprüft. Die Ermittlung von Umsatz, Ausbeuten und Selektivitäten erfolgte mittels GC/MS.

**Tabelle 2**

| Beispiel | Phosphorkomponente | Phasentransferkatalysator | t [min] | Umsatz von CHDD [%] | Ausbeute an **I** [%] | Selektivität zu **I** [%] |
|---|---|---|---|---|---|---|
| 16 | (C₆H₅)₂P(O)OH | [CH₃(C₈H₁₇)₃N]HSO₄ | 20 | 0,2 | 0,2 | 99,0 |
| | | | 40 | 2,0 | 2,0 | 99,0 |
| | | | 60 | 3,2 | 3,1 | 99,0 |
| | | | 80 | 6,2 | 6,1 | 99,0 |
| | | | 100 | 11,7 | 11,3 | 96,6 |
| | | | 120 | 16,2 | 15,6 | 95,9 |
| | | | 180 | 24,1 | 22,4 | 93,0 |
| | | | 240 | 28,6 | 26,0 | 90,9 |

### Vorschrift für Beispiel 17 (Tabelle 3)

Na₂WO₄ (0,165 g, 0,50 mmol), Aminomethanphosphonsäure (0,50 mmol) und Methyltrioctylammoniumhydrogensulfat (0,233 g, 0,50 mmol) wurden in einen 50-ml-Dreihalskolben gegeben. 1,9-Cyclohexadecadien (Isomerengemisch, 25 mmol, 5,51 g), H₂O (5,00 ml) und 1,2-Dichlorethan (20,00 ml) wurden anschließend hinzugefügt. Es bildeten sich zwei Phasen, eine organische Phase, welche aus 1,2-Dichlorethan und CHDD bestand, und eine wässrige Phase, welche die Präkursoren für den Katalysator enthielt. Das Gemisch wurde dann bei 700 rpm gerührt und auf die Reaktionstemperatur von 60 °C aufgeheizt. Als diese Temperatur erreicht war, wurde die erste Portion H₂O₂ (50 Gew.%) (1.10 g, 16,2 mmol, 0,65 Moläqu.) zugegeben und die Reaktion gestartet. Nach 30 und 60 min wurde jeweils eine weitere Portion H₂O₂ zugetropft (1.10 g, 16,2 mmol, 0,65 Moläqu. pro Portion). Danach wurde noch 1,5 Stunden bei 60 °C weiter gerührt. Der Fortgang der Reaktion wurde durch Probennahme aus der organischen Phase alle 20 Minuten während der ersten 100 Minuten und am Ende des Experiments überprüft. Die Ermittlung von Umsatz, Ausbeuten und Selektivitäten erfolgte mittels GC/MS.

**Tabelle 3**

| Beispiel | Phosphorkomponente | Phasentransferkatalysator | t [min] | Umsatz von CHDD [%] | Ausbeute an **I** [%] | Selektivität zu **I** [%] |
|---|---|---|---|---|---|---|
| 17 | H₂NCH₂P(O)(OH)₂ | [CH₃(C₈H₁₇)₃N]HSO₄ | 20 | 5,3 | 5,3 | 99,0 |
| | | | 40 | 12,4 | 12,4 | 99,0 |
| | | | 60 | 25,1 | 23,5 | 93,5 |
| | | | 80 | 36,6 | 33,4 | 91,3 |
| | | | 100 | 44,9 | 40,0 | 89,0 |

### Vorschrift für Beispiel 18 (Tabelle 4)

H₂WO₄ (0,125 g, 0,50 mmol), Phosphorsäure (0,50 mmol) und Methyltrioctylammoniumhydrogensulfat (0,233 g, 0,50 mmol) wurden in einen 50-ml-Dreihalskolben gegeben. 1,9-Cyclohexadecadien (Isomerengemisch, 25 mmol, 5,51 g), H₂O (5,00 ml) und Toluen (20,00 ml) wurden anschließend hinzugefügt. Es bildeten sich zwei Phasen, eine organische Phase, welche aus Toluen und CHDD bestand, und eine wässrige Phase, welche die Präkursoren für den Katalysator enthielt. Das Gemisch wurde dann bei 700 rpm gerührt und auf die Reaktionstemperatur von 60 °C aufgeheizt. Als diese Temperatur erreicht war, wurde die erste Portion H₂O₂ (50 Gew.%) (0.74 g, 10,9 mmol, 0,43 Moläqu.) zugegeben und die Reaktion gestartet. Nach 30 und 60 min wurde jeweils eine weitere Portion H₂O₂ zugetropft (0.74 g, 10,9 mmol, 0,43 Moläqu. pro Portion). Danach wurde noch 1,5 Stunden bei 60 °C weiter gerührt. Der Fortgang der Reaktion wurde durch Probennahme aus der organischen Phase alle 20 Minuten während der ersten 100 Minuten und am Ende des Experiments überprüft. Die Ermittlung von Umsatz, Ausbeuten und Selektivitäten erfolgte mittels GC/MS.

**Tabelle 4**

| Beispiel | Phosphorkomponente | Phasentransferkatalysator | t [min] | Umsatz von CHDD [%] | Ausbeute an **I** [%] | Selektivität zu **I** [%] |
|---|---|---|---|---|---|---|
| 18 | H₃PO₄ | [CH₃(C₈H₁₇)₃N]HSO₄ | 20 | 24,7 | 24,7 | 99,0 |
| | | | 40 | 44,0 | 41,6 | 94,5 |
| | | | 60 | 57,2 | 50,6 | 88,4 |

### Vorschrift für Beispiel 19 (Tabelle 5)

Na₂WO₄ (0,165 g, 0,50 mmol), Hydroxymethanphosphonsäure (0,50 mmol) und Methyltrioctylammoniumhydrogensulfat (0,50 mmol) wurden in einen 25-ml-Dreihalskolben gegeben. 1,9-Cyclohexadecadien (Isomerengemisch, 25 mmol, 5,51 g) und H₂O (5,00 g) wurde anschließend hinzugefügt. Es bildeten sich zwei Phasen, eine organische Phase, welche aus CHDD bestand, und eine wässrige Phase, welche die Präkursoren für den Katalysator enthielt. Das Gemisch wurde dann bei 800 rpm gerührt und auf die Reaktionstemperatur von 60 °C aufgeheizt. Als diese Temperatur erreicht war, wurde die erste Portion H₂O₂ (50 Gew.%) (0,47 g, 6,91 mmol, 0,27 Moläqu.) zugegeben und die Reaktion gestartet. Nach 60 min wurde eine zweite Portion H₂O₂ zugetropft (0.47 g, 6,91 mmol, 0,27 Moläqu.). Danach wurde noch 2 Stunden bei 60 °C weiter gerührt. Der Fortgang der Reaktion wurde durch Probennahme aus der organischen Phase alle 20 Minuten während der ersten zwei Stunden und am Ende des Experiments überprüft. Die Ermittlung von Umsatz, Ausbeuten und Selektivitäten erfolgte mittels GC/MS.

**Tabelle 5**

| Beispiel | Phosphorkomponente | Phasentransferkatalysator | t [min] | Umsatz von CHDD [%] | Ausbeute an **I** [%] | Selektivität zu **I** [%] |
|---|---|---|---|---|---|---|
| 19 | HOCH₂P(O)(OH)₂ | [CH₃(C₈H₁₇)₃N]HSO₄ | 40 | 13,6 | 13,6 | 99,0 |
| | | | 100 | 28,5 | 27,0 | 94,9 |
| | | | 120 | 36,5 | 32,6 | 89,4 |

### Vorschrift für Beispiel 20 (Tabelle 6)

Na₂WO₄ (0,083 g, 0,25 mmol), Bis(hydroxymethan)phosphinsäure (0,031 g, 0,25 mmol) und Methyltrioctylammoniumhydrogensulfat (0,25 mmol) wurden in einen 25-ml-Dreihalskolben gegeben. 1,9-Cyclohexadecadien (Isomerengemisch, 12,5 mmol, 2,75 g), H₂O (2,50 g) und Toluen (10,00 g) wurden anschließend hinzugefügt. Es bildeten sich zwei Phasen, eine organische Phase, welche aus Toluen und CHDD bestand, und eine wässrige Phase, welche die Präkursoren für den Katalysator enthielt. Das Gemisch wurde dann bei 800 rpm gerührt und auf die Reaktionstemperatur von 60 °C aufgeheizt. Als diese Temperatur erreicht war, wurde die erste Portion H₂O₂ (50 Gew.%) (0,24 g, 3,53 mmol, 0,28 Moläqu.) zugegeben und die Reaktion gestartet. Nach 60 min wurde eine zweite Portion H₂O₂ zugetropft (0,24 g, 3,53 mmol, 0,28 Moläqu.). Danach wurde noch 2 Stunden bei 60 °C weiter gerührt. Der Fortgang der Reaktion wurde durch Probennahme aus der organischen Phase alle 20 Minuten während der ersten zwei Stunden und am Ende des Experiments überprüft. Die Ermittlung von Umsatz, Ausbeuten und Selektivitäten erfolgte mittels GC/MS.

**Tabelle 6**

| Beispiel | Phosphorkomponente | Phasentransferkatalysator | t [min] | Umsatz von CHDD [%] | Ausbeute an **I** [%] | Selektivität zu **I** [%] |
|---|---|---|---|---|---|---|
| 20 | (CH₂OH)₂P(O)OH | [CH₃(C₈H₁₇)₃N]HSO₄ | 20 | 2,5 | 2,5 | 99 |
| | | | 40 | 6,3 | 6,3 | 99 |
| | | | 60 | 11,3 | 11,3 | 99 |
| | | | 80 | 20,8 | 20,5 | 98,6 |
| | | | 100 | 29,2 | 28,4 | 97,3 |
| | | | 120 | 32,4 | 30,7 | 94,9 |
| | | | 180 | 45,7 | 40,8 | 89,3 |

### Vorschrift für Beispiel 21 (Tabelle 7)

Na₂WO₄ (0,083 g, 0,25 mmol), Bis(hydroxymethan)phosphinsäure (0,031 g, 0,25 mmol) und Methyltrioctylammoniumhydrogensulfat (0,25 mmol) wurden in einen 25-ml-Dreihalskolben gegeben. 1,9-Cyclohexadecadien (Isomerengemisch, 12,5 mmol, 2,75 g), H₂O (2,50 g) und Toluen (10,00 g) wurden anschließend hinzugefügt. Es bildeten sich zwei Phasen, eine organische Phase, welche aus Toluen und CHDD bestand, und eine wässrige Phase, welche die Präkursoren für den Katalysator enthielt. Das Gemisch wurde dann bei 800 rpm gerührt und auf die Reaktionstemperatur von 80 °C aufgeheizt. Als diese Temperatur erreicht war, wurde die erste Portion H₂O₂ (50 Gew.%) (0,24 g, 3,53 mmol, 0,28 Moläqu.) zugegeben und die Reaktion gestartet. Nach 60 min wurde eine zweite Portion H₂O₂ zugetropft (0,24 g, 3,53 mmol, 0,28 Moläqu.). Danach wurde noch 2 Stunden bei 80 °C weiter gerührt. Der Fortgang der Reaktion wurde durch Probennahme aus der organischen Phase alle 20 Minuten während der ersten zwei Stunden und am Ende des Experiments überprüft. Die Ermittlung von Umsatz, Ausbeuten und Selektivitäten erfolgte mittels GC/MS.

**Tabelle 7**

| Beispiel | Phosphorkomponente | Phasentransferkatalysator | t [min] | Umsatz von CHDD [%] | Ausbeute an **I** [%] | Selektivität zu **I** [%] |
|---|---|---|---|---|---|---|
| 21 | (CH₂OH)₂P(O)OH | [CH₃(C₈H₁₇)₃N]HSO₄ | 20 | 12,2 | 12,1 | 99,0 |
| | | | 60 | 27,1 | 26,4 | 97,2 |
| | | | 80 | 39,7 | 35,2 | 88,5 |
| | | | 100 | 45,5 | 39,4 | 86,5 |
| | | | 120 | 47,4 | 38,9 | 82,1 |

## Patentansprüche

1. Verfahren zur Herstellung von 17-Oxabicyclo[14.1.0]heptadec-8-en, umfassend eine Reaktion mit den Reaktanten Cyclohexadeca-1,9-dien und Wasserstoffperoxid, worin die Reaktion in Gegenwart eines Katalysators durchgeführt wird.

2. Verfahren nach Anspruch 1, **dadurch gekennzeichnet, dass** die Reaktion in einem Zweiphasensystem durchgeführt wird.

3. Verfahren nach einem der vorherigen Ansprüche, **dadurch gekennzeichnet, dass** der Katalysator Phosphor enthält.

4. Verfahren nach einem der vorherigen Ansprüche, **dadurch gekennzeichnet, dass** der Katalysator Wolfram enthält.

5. Verfahren nach einem der vorherigen Ansprüche, **dadurch gekennzeichnet, dass** die aktive Spezies des Katalysators ein Peroxowolframatophosphat enthält.

6. Verfahren nach einem der vorherigen Ansprüche, **dadurch gekennzeichnet, dass** die aktive Spezies des Katalysators das Anion {PO₄[WO(O₂)₂]₄}³⁻ enthält.

7. Verfahren nach einem der vorherigen Ansprüche, **dadurch gekennzeichnet, dass** die aktive Spezies des Katalysators ein Kation eines Phasentransferkatalysators, bevorzugt ein Tetraalkylammoniumkation, enthält.

8. Verfahren nach einem der vorherigen Ansprüche, **dadurch gekennzeichnet, dass** die aktive Spezies des Katalysators ein Kation eines Phasentransferkatalysators der Formel
R¹ₙR²ₘN⁺
enthält, **dadurch gekennzeichnet, dass**
R¹ und R² jeweils C1-C30 n-Alkyl bedeuten und R¹ gleich oder verschieden zu R² ist,
sowie die Summe aus m und n gleich 4 ist.

9. Verfahren nach einem der vorherigen Ansprüche, **dadurch gekennzeichnet, dass** die aktive Spezies des Katalysators aus mindestens einer phosphorenthaltenden Säure, mindestens einer Wolfram (VI)-Verbindung und mindestens einem Phasentransferkatalysator, bevorzugt in situ, gebildet wird.

10. Verfahren nach Anspruch 9, **dadurch gekennzeichnet, dass** die phosphorenthaltende Säure ausgewählt wird aus Phosphorsäure, Phosphonsäuren, Phosphinsäuren sowie Heteropolysäuren und deren Derivaten,
die Wolfram(VI)-verbindung ausgewählt wird aus Alkaliwolframaten, Erdalkaliwolframaten, Ammoniumwolframaten oder Wolframtrioxid-monohydrat, bevorzugt Natriumwolframat,
oder/und
der Phasentransferkatalysator ausgewählt wird aus einem Tetraalkylammoniumsalz, bevorzugt einer Verbindung der Formel
(R¹ₙR²ₘN⁺)_{y}X^{y-},
**dadurch gekennzeichnet, dass**
R¹ und R² jeweils C1-C30 n-Alkyl bedeuten und R¹ gleich oder verschieden zu R² ist, X^{y-} gleich Cl⁻, Br⁻, J⁻, HSO₄⁻, SO₄²⁻, H₂PO₄⁻, HPO₄²⁻, PO₄³⁻, CH₃SO₃⁻, CF₃SO₃⁻, CH₃C₆H₄SO₃⁻, ClO₃⁻, ClO₄⁻, oder NO₃⁻ bedeutet, und die Summe aus m und n gleich 4 ist und y gleich 1, 2 oder 3 ist.

11. Verwendung einer wässrigen Mischung als Katalysatorpräkursor in einem Verfahren nach einem der vorherigen Ansprüche, umfassend mindestens eine phosphorenthaltende Säure, mindestens eine Wolfram (VI)-Verbindung und mindestens einen Phasentransferkatalysator.

12. Verwendung einer Verbindung der Formel
[R¹ₙR²ₘN⁺]₃{PO₄[WO(O₂)₂]₄},
**dadurch gekennzeichnet, dass** R¹ und R² jeweils C1-C30 n-Alkyl bedeuten und R¹ gleich oder verschieden zu R² ist, sowie die Summe aus m und n gleich 4 ist, als aktive Spezies eines Katalysators in einem Verfahren nach einem der
vorherigen Ansprüche.

## Claims

1. Process for producing 17-oxabicyclo[14.1.0]heptadec-8-ene, comprising a reaction involving the reactants cyclohexadeca-1,9-diene and hydrogen peroxide, in which the reaction is carried out in the presence of a catalyst.

2. Process according to claim 1, **characterized in that** the reaction is carried out in a two-phase system.

3. Process according to one of the preceding claims, **characterized in that** the catalyst contains phosphorus.

4. Process according to one of the preceding claims, **characterized in that** the catalyst contains tungsten.

5. Process according to one of the preceding claims, **characterized in that** the active species of the catalyst contains a peroxotungstophosphate.

6. Process according to one of the preceding claims, **characterized in that** the active species of the catalyst contains the anion {PO₄[WO(O₂)₂]₄}³⁻.

7. Process according to one of the preceding claims, **characterized in that** the active species of the catalyst contains a cation of a phase transfer catalyst, preferably a tetraalkylammonium cation.

8. Process according to one of the preceding claims, **characterized in that** the active species of the catalyst contains a cation of a phase transfer catalyst of the formula
R¹ₙR²ₘN⁺,
**characterized in that**
R¹ and R² each represent C1-C30 n-alkyl and R¹ is identical to or different from R²,
and the sum of m and n is equal to 4.

9. Process according to one of the preceding claims, **characterized in that** the active species of the catalyst is formed, preferably in situ, from at least one phosphorus-containing acid, at least one tungsten(VI) compound and at least one phase transfer catalyst.

10. Process according to claim 9, **characterized in that** the phosphorus-containing acid is selected from phosphoric acid, phosphonic acids, phosphinic acids and heteropoly acids, and derivatives thereof,
the tungsten(VI) compound is selected from alkali tungstates, alkaline-earth tungstates, ammonium tungstates, or tungsten trioxide monohydrate, preferably sodium tungstate,
or/and
the phase transfer catalyst is selected from a tetraalkylammonium salt, preferably a compound of the formula
(R¹ₙR²ₘN⁺)_{y}X^{y-},
**characterized in that**
R¹ and R² each represent C1-C30 n-alkyl and R¹ is identical to or different from R², X^{y-} represents Cl⁻, Br, I⁻, HSO₄⁻, SO₄²⁻, H₂PO₄⁻, HPO₄²⁻, PO₄³⁻, CH₃SO₃⁻, CF₃SO₃⁻, CH₃C₆H₄SO₃⁻, ClO₃⁻, ClO₄⁻ or NO₃⁻, and the sum of m and n is equal to 4 and y is equal to 1, 2 or 3.

11. Use of an aqueous mixture as a catalyst precursor in a process according to one of the preceding claims, comprising at least one phosphorus-containing acid, at least one tungsten(VI) compound and at least one phase transfer catalyst.

12. Use of a compound of the formula
[R¹ₙR²ₘN⁺]₃{PO₄[WO(O₂)₂]₄},
**characterized in that** R¹ and R² each represent C1-C30 n-alkyl and R¹ is identical to or different from R², and the sum of m and n is equal to 4, as an active species of a catalyst in a process according to one of the preceding claims.

## Revendications

1. Procédé de préparation de 17-oxabicyclo[14.1.0]heptadec-8-ène, comprenant une réaction avec les réactifs cyclohexadéca-1,9-diène et peroxyde d'hydrogène, la réaction ayant lieu en présence d'un catalyseur.

2. Procédé selon la revendication 1, **caractérisé en ce que** la réaction a lieu dans un système à deux phases.

3. Procédé selon l'une des revendications précédentes, **caractérisé en ce que** le catalyseur contient du phosphore.

4. Procédé selon l'une des revendications précédentes, **caractérisé en ce que** le catalyseur contient du tungstène.

5. Procédé selon l'une des revendications précédentes, **caractérisé en ce que** l'élément actif du catalyseur contient un phosphate peroxytungstate.

6. Procédé selon l'une des revendications précédentes, **caractérisé en ce que** l'élément actif du catalyseur contient l'anion {PO₄[WO(O₂)₂]₄}³⁻.

7. Procédé selon l'une des revendications précédentes, **caractérisé en ce que** l'élément actif du catalyseur contient un cation d'un catalyseur de transfert de phase, préférentiellement un cation de tétraalkylammonium.

8. Procédé selon l'une des revendications précédentes, **caractérisé en ce que** l'élément actif du catalyseur contient un cation d'un catalyseur de transfert de phase de formule
R¹ₙR²ₘN⁺
**caractérisé en ce que**
R¹ et R² signifient chacun C1-030 n-alkyle et R¹ est identique à R² ou différent de celui-ci,
et que la somme de m et n est égale à 4.

9. Procédé selon l'une des revendications précédentes, **caractérisé en ce que** l'élément actif du catalyseur est constitué d'au moins un acide contenant du phosphore, d'au moins un composé de tungstène (VI) et d'au moins un catalyseur de transfert de phase, préférentiellement in situ.

10. Procédé selon la revendication 9, **caractérisé en ce que** l'acide contenant du phosphore est sélectionné entre acide phosphorique, acides phosphoniques, acides hypophosphoneux ainsi qu'hétéropolyacides et leurs dérivés,
le composé de tungstène (VI) est sélectionné entre tungstates alcalins, tungstates alcalino-terreux, tungstates d'ammonium ou trioxyde de tungstène monohydraté, préférentiellement tungstate de sodium,
et/ou
le catalyseur de transfert de phase est sélectionné entre un sel de tétraalkylammonium, préférentiellement un composé de formule
(R¹ₙR²ₘN⁺)_{y}X_{y}⁻,
**caractérisé en ce que**
R¹ et R² représentent chacun un n-alkyle C1-C30 et R¹ est identique à R² ou différent de celui-ci, Xy⁻ représente Cl⁻, Br, J⁻, HSO₄⁻, SO₄²⁻, H₂PO₄⁻, HPO₄²⁻, PO₄³⁻, CHSO₃⁻, CF₃SO₃⁻, CH₃C₆H₄SO₃⁻, ClO₃⁻, CLO₄⁻ ou NO₃, et la somme de m et n est égale à 4 et y est égale à 1, 2 ou 3.

11. Utilisation d'un mélange aqueux comme précurseur de catalyseur dans un procédé selon l'une des revendications précédentes, comprenant au moins un acide contenant du phosphore, au moins un composé de tungstène (VI) et au moins un P catalyseur de transfert de phase.

12. Utilisation d'un composé de formule
[R¹nR²mN⁺]₃{PO₄[WO(O₂)₂]₄},
**caractérisé en ce que** R¹ et R² représentent chacun un n-alkyle C1-C30 et R¹ est identique à R² ou différent de celui-ci, et **en ce que** la somme de m et n est égale à 4, comme élément actif d'un catalyseur dans un procédé selon l'une des revendications précédentes.
